# EUROPEAN PATENT APPLICATION

(11) **EP 1 733 793 A2**
(43) Date of publication of application: **20.12.2006**
(21) Application number: 06253111.6
(22) Date of filing: 15.06.2006
(51) Int. Cl.: B01L 3/00, C12M 1/20

(54) **Multi-well filter plate with shifted wells and U-bottom receiver plate**

(30) Priority: 16.06.2005 US 154171
(71) Applicant: MILLIPORE CORPORATION, Billerica, Massachusetts 01821 (US)
(72) Inventor: Desilets, Kenneth G., Westfor, Massachusetts 01886 (US); Scott, Christopher A., Westford, Massachusetts 01886 (US)
(74) Representative: Greenwood, John David

(57) **Abstract**

Multiwell filter comprising a filter plate having a plurality of wells, each including a support. Each well has a corresponding adjacent access port providing access to the corresponding feeding/receiver plate well, e.g. via a pipette. When the filter plate is positioned over a feeding/receiver plate having a plurality of wells, each well of the feeding/receiving plate aligns with and communicates with a corresponding filter plate well. Each access port provides access to a respective well in the feeding/receiver plate, and the wells in the filter plate are not centrally located with respect to the feeding/receiver plate wells, but are shifted in the x and y directions relative to the receiver wells in an amount effective for reducing or eliminating capillary holdup in the gap between the filter wells and the feeding/receiver wells. The wells in the feeding/receiver plate preferably are formed in a U-shaped bottom below each access port.

## Description

### FIELD OF THE INVENTION

The present invention relates to apparatus for transport or permeation testing, such as culturing cells onto a membrane and then using the membrane as a substrate to simulate an epithelial cell layer to carry out transport or permeation testing thereon. In particular, the present invention relates to a multiwell filter device which allows for adding or removing liquid from the feeding tray of the apparatus without disturbing a material such as cells contained within the wells.

### BACKGROUND OF THE INVENTION

It is often desirable to evaluate how various chemicals that are orally ingested into a human will be absorbed into the blood stream through the intestinal wall. Such evaluation can be useful in, for example, drug testing to determine how various drugs will be absorbed into the blood stream. Transport of various substances through other types of epithelial cell layers can also be useful in therapeutically treating patients.

The MultiScreen® Caco-2 96-well device commercially available from Millipore Corporation is designed to support attachment, growth and differentiation of Caco-2 and other adherent cell lines. After the formation of a differentiated Caco-2 cell monolayer, the device can then be used to measure the rate of known and unknown drug transport across the Caco-2 cell barrier. All procedures are designed to be carried out in a single device and can be performed using automation for cell seeding, cell feeding, washing and drug transport experiments. Caco-2 cells possess many of the properties of the small intestine. As such, these cells represent a useful and well-accepted tool for studying the absorption and/or secretion of drugs/chemicals across the intestinal mucosa.

The MultiScreen® Caco-2 device is designed to support adherent cell growth and differentiation, particularly the ATCC^{®} cell line Caco-2 designated as HTB-37. These cells are ideally suited for the performance of drug absorption experiments to determine the rate at which a known or unknown drug will penetrate an intestinal cell barrier. The MultiScreen Caco-2 device consists of a filter plate with 96 basolateral access holes, receiver plate, lid and additional receiver plate for analysis. It is ANSI-SBS-compliant; the Caco-2 device is compatible with automated liquid handling systems and span pipettes. An example of such a device is shown in WO 02/102962 assigned to Millipore Corporation, the disclosure of which is hereby incorporated by reference.

Extraction of most or all of the media from the feeding/receiver plate of a multi-well device is an important factor in the overall functionality and efficiency of the device. In addition, a reduction in the volume under the filter plate will allow the use of less media to feed the cells, which can be a substantial cost advantage.

It is therefore an object of the present invention to provide a multi-well device that includes a feeding/receiver plate having a U-shaped configuration under the active filter area.

It is a further object of the present invention to provide a multi-well filtration device wherein the relative positioning of the filter plate wells and the feeding/receiver plate wells is shifted in order to reduce or eliminate capillary hold-up.

### SUMMARY OF THE INVENTION

The problems of the prior art have been overcome by the present invention, which provides a multiwell filter device comprising a filter plate having a plurality of wells, each well including a support such as a membrane for retaining sample. Preferably each well has a corresponding adjacent access port, which provides direct access to the bottom of the corresponding feeding/receiver plate well, such as via a pipette tip inserted into the access port. The filter plate is configured to be positioned over a feeding/receiver plate having a plurality of wells, such that each respective well of the plurality of wells of the feeding/receiving plate aligns with and is communication with a corresponding well of the filter plate. Moreover, the alignment is such that each of the access ports in the filter plate provides access to a respective well in the feeding/receiver plate. However, unlike the prior art, when the filter plate is positioned over the feeding/receiver plate, the wells in the filter plate are not centrally located with respect to the wells in the feeding/receiver plate. Instead, the wells in the filter plate are shifted in the x and y directions relative to the feeding/receiver wells an amount effective for reducing or eliminating capillary holdup of liquid in the gap between the filter wells and the feeding/receiver wells.

In a further embodiment of the present invention, the wells in the feeding/receiver plate are designed to create a U-shaped bottom below each access port to provide a more uniform volume of feeding media and allow for improved medial removal through the access port, such as via syringe or pipette.

### BRIEF DESCRIPTION OF THE DRAWINGS

Figure 1 is a top view of a filter plate in accordance with the present invention;
Figure 2 is a side view of a filter plate positioned over a feeding/receiver plate in accordance with the present invention;
Figure 3 is a cross-sectional view of the filter plate positioned over the feeding/receiver plate along line A―A of Figure 1;
Figure 4 is a cross-sectional view of the filter plate positioned over the feeding/receiver plate along line B―B of Figure 1;
Figure 5 is a cross-sectional view of the filter plate positioned over the feeding/receiver plate along line X-X of Figure 1;
Figure 6A is a cross-sectional view of a filter plate positioned over a feeding/receiver plate in accordance with the prior art, showing capillary hold-up of liquid;
6B is a cross-sectional view of the filter plate positioned over the feeding/receiver plate along line C―C of Figure 1 in accordance with the present invention;
Figure 7 is a cross-sectional view of the filter plate positioned over the feeding/receiver plate with a pipette shown accessing a receiver plate well;
Figure 8a is a top view of a feeding/receiver plate well of the prior art; and
Figure 8b is a top view of the feeding/receiver plate well in accordance with the present invention.

### DETAILED DESCRIPTION OF THE INVENITON

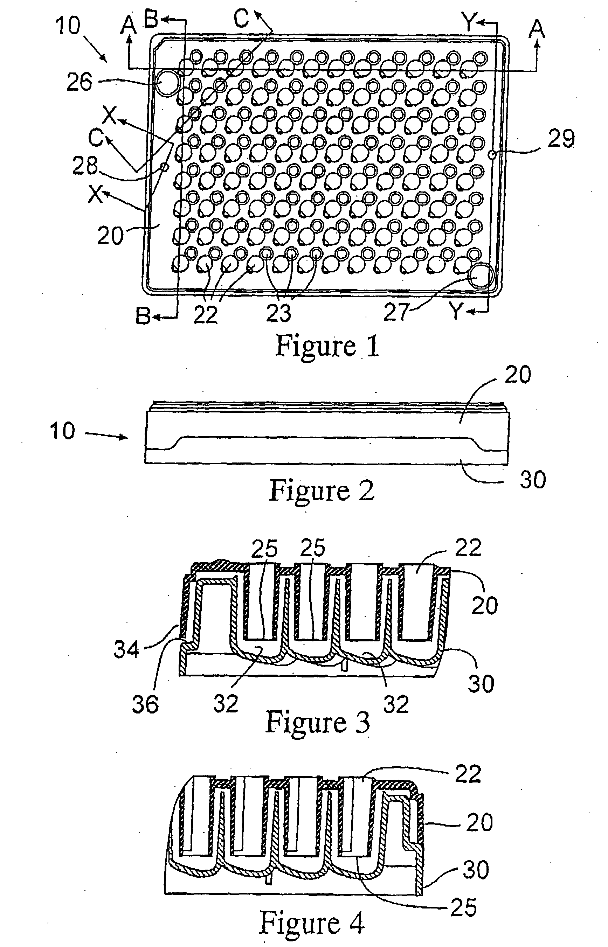

Turning first to Figures 1 and 2, there is shown a multiwell device 10 comprising a filter plate 20 including a plurality of wells 22. Preferably an access port 23 is adjacent to each well 22 as shown. In the embodiment shown, there are 96 wells 22 and 96 access ports 23, configured in a 8 x 12 array, although those skilled in the art will appreciate that fewer or more wells (and access ports) could be employed. Each of the wells 22 is preferably tubular, preferably tapering to a slightly narrower bottom as best seen in Figure 3. A support, preferably a microporous membrane 25, is secured at the bottom of each well 22 and is preferably sealed thereto by means known in the art. The area of the membrane that is in communication with the well 22 is the active filtration area. Access holes 26, 27 can be located on the filter plate 20 for adding or removing liquid nutrient from the device, especially when used with a separate single well feeding tray.

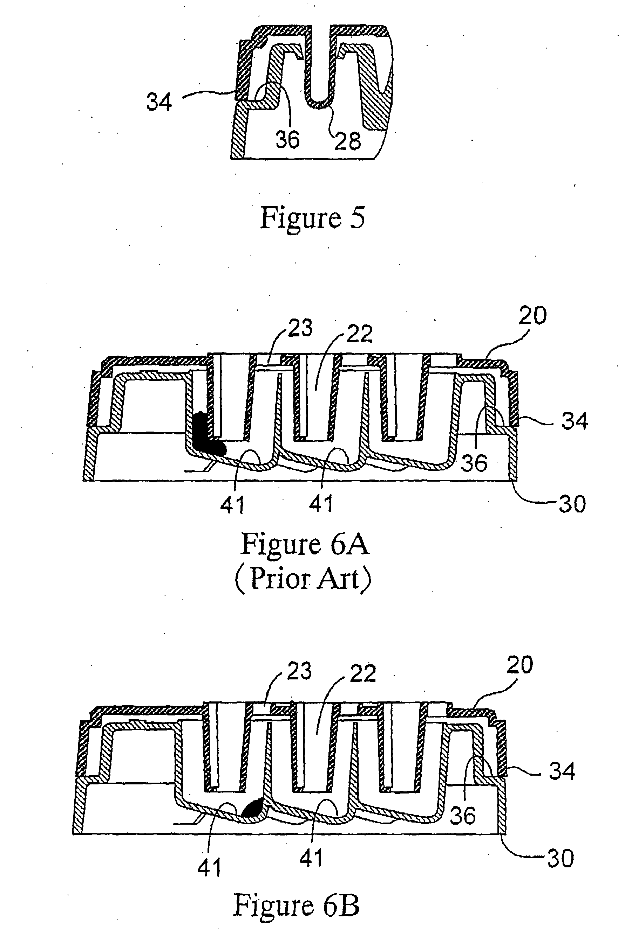

The filter plate 20 is configured to be positioned over and aligned with a feeding/receiver plate 30 (hereinafter referred to as a receiver plate, although those skilled in the art will appreciate that the plate can function as a feeding plate. as well). Suitable posts, cones, pyramids, pins, dimples or similar alignment mechanisms 28 (Figure 5) can be provided on the filter plate 20 that can be positioned in corresponding holes in the receiver plate to effectuate and maintain the proper relative alignment. Preferably the members 28 are offset or different from one another so that the filter plate can be positioned on the receiver plate in only one configuration. Those skilled in the art will appreciate that the alignment mechanisms can be reversed, whereby the posts, cones, pyramids, etc. can be formed in the receiver plate and corresponding holes in the filter plate. The filter plate 20 preferably also includes four legs 34, one at each corner, which extend lower than the sides of the plate and are shaped to sit on shoulders 36 of the receiver plate 30 as best seen in Figures 3 and 5. The legs 34 add to the stability of the filter plate 20 when positioned over the receiver plate 30, and also serve as a vertical stop, preventing further penetration of the filter plate wells 22 into the receiver plate wells 32, which further penetration could cause each membrane 25 to contact the bottom of a well 32 and damage the membrane or deleteriously interfere with the assay.

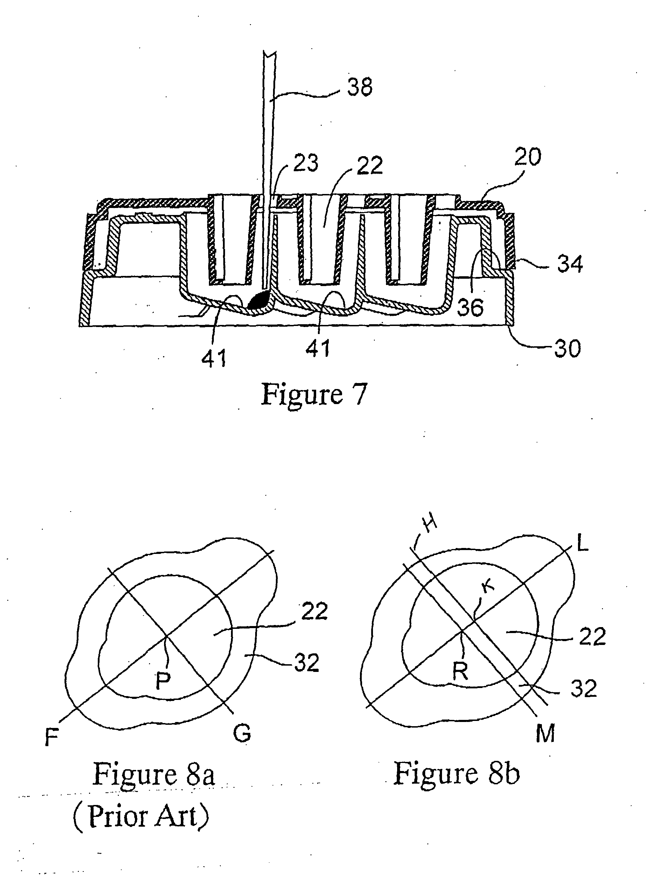

When the filter plate 20 is properly positioned over the receiver plate 30, each well 22 of the filter plate 20 extends into a corresponding well 32 of the receiver plate 30, ensuring that each membrane 25 extends into only one receiver well 32. Accordingly, the inside diameter of each receiver plate well 32 is larger than the largest outside diameter of each filter plate well 22 in order to enable each filter plate well 22 to enter into and reside a respective receiver plate well as seen in Figures 3 and 4, for example. A gap (annular in the embodiment shown) is thus formed between the outside surface of each well 22 and the inside surface of each well 32. As best seen in Figure 7, a portion of this gap provides access to the receiver well 32, such as by pipette 38, via the corresponding access port 23 associated with a given well 22.

In accordance with a preferred embodiment of the present invention, when the filter plate 20 is properly positioned on the receiver plate 30, the relative positioning of the filter plate wells 22 and receiver plates wells 32 is shifted from the conventional centered positioning in order to reduce or eliminate capillary hold-up between the interior wall of each receiver plate well and the exterior wall of each respective filter plate well (an example of such capillary hold-up is illustrated in Figure 6A). More specifically, the wells of either the filter plate or the receiver plate (or both) are shifted from conventional positioning, in the x and y-directions, in order to increase the gap between each receiver well wall and corresponding filter plate well in the area opposite each access port 23 when the filter plate and receiver plate are in the assembled, engaged relationship, as shown in Figure 6B. This is the area where capillary hold-up typically had been most problematic. Those skilled in the art will appreciate that the shift in well positioning is a relative shift between the filter plate wells 22 and receiver plate wells 32, so that an alterative embodiment maintains the filter plate wells 22 in the conventional position and shifts the receiver plate wells 32 accordingly. Although the relative shift is sufficient to reduce and preferably eliminate capillary hold-up, it is not so significant as to impede access to fluid in the receiver plate wells 32 via access ports 23, as can be seen in Figure 7.

Since the shape (as seen from a cross-sectional top view in Figure 8b) of the receiver wells 32 is not uniform, the relative shift for any shaped well can be described as follows. The reference point in each receiver plate well is the intersection of the longest axis drawn through a top cross-section (i.e., in a horizontal plane) of the well and the longest axis perpendicular to it. Thus, in the embodiment of Figure 8b, L is the longest axis drawn through the well 32, and M is the longest axis drawn perpendicularly to it. The two axes intersect at reference point R. H is the longest axis drawn through the top cross-section (i.e., in a horizontal plane) of filter plate well 22 drawn perpendicularly to line L. Lines H and L intersect at reference point K. In accordance with the present teachings, reference point R of a receiver well 32 and reference point K of a corresponding filter plate well 22 do not align; they do not share the same vertical axis that is perpendicular to horizontal due to the shift. In contrast, Figure 8a depicts a filter well 22 and receiver plate well 32 aligned as in the prior art, where F is the longest axis drawn through the active filter area of the well 22 and through receiver plate well 32, and G is the longest axis drawn perpendicularly to axis F. There is no offset.

Suitable shapes for the receiver wells 32 include tear drops, ovals, diamonds, squares, rectangles, circles, regular polygons, irregular polygons, etc.

In a further embodiment of the present invention, the wells 32 of the receiver plate 30 have a U-shaped bottom below the active filter area, as best seen in Figures 3, 4 and 6B. The deepest portion 41 of the U-shaped bottom is located below the access port 23, preferably at the removal point of the liquid. Thus, the U-shaped bottom is defined by uniformly downwardly sloping walls, terminating in a nadir located below the access port, preferably at a corner of each receiver plate well 32. At every cross-section of the receiver well 32, the U-shaped bottom portion 41 is the lowest point, and thus there is never a linear cross-section between the high point and low point of the well.

The volume under the filter area can be smaller (e.g., less than 10 microliters of sample remains after removal by pipette) with a U-shaped bottom draining to a U-shaped aspirating point at 41, and less media can be used to feed the cells. Moreover, a more uniform volume of feeding media to growing cells results, which allows for more uniform cell growth, compared to a slanted bottom which results in a varying volume and cell growth. The U-shape at the liquid removal point offers an improved amount of removal of the media.

Despite the relative positioning of the filter plate and feeding plate wells, the device remains automation compliant (e.g., complaint with ANSI-SBS standards).

## Claims

1. Multi-well filtration apparatus, comprising:
a filter plate having a plurality of filter plate wells, each of said wells having a support sealed to an end thereof, and a plurality of access holes such that each of said plurality of wells has an associated access hole;
a feeding or receiver plate having a plurality of feeding or receiver plate wells, each of said feeding or receiver plate wells being in fluid communication with a respective filter plate well and access port when said filter plate is properly aligned over said feeding or receiver plate;
wherein the relative positioning of said filter plate wells and said feeding or receiver plate wells is shifted such that intersection of the longest axis drawn through a top cross-section of each feeding or receiver plate well and the longest axis perpendicular to it does not vertically align with the intersection of the longest axis drawn through a top cross-section of the active filter area of each filter plate well and the longest axis drawn perpendicularly to it.

2. The apparatus of claim 1, wherein each feeding or receiver plate well has a U-shaped bottom, such that there is never a linear cross-section between the high point and low point of the well.

3. The apparatus of claim 1, wherein said filter plate comprises 96 wells.

4. The apparatus of claim 1, wherein the overall dimensions of said filter plate and said feeding or receiving plate are automation compliant.

5. The apparatus of claim 1, wherein said support comprises a membrane.

6. The apparatus of claim 5, wherein said membrane is microporous.

7. The apparatus of claim 1, wherein said support comprises a permeable barrier.

8. The apparatus of claim 2, wherein said U-shaped bottom has a deepest portion in vertical alignment with a respective access port.

9. Multi-well filtration apparatus, comprising:
a filter plate having a plurality of filter plate wells, each of said wells having an inlet, a support sealed to an end thereof, and a plurality of access holes such that each of said plurality of wells has an associated access hole;
a feeding or receiver plate having a plurality of feeding or receiver plate wells, each of said feeding or receiver plate wells being in fluid communication with a respective filter plate well and access port when said filter plate is properly aligned over said feeding or receiver plate, each of said feeding or receiving plate wells having a deepest portion relative to a respective filter plate well inlet, said deepest portion being positioned below said associated access port 23.

10. The multi-well filtration apparatus of claim 9, wherein each of said feeding or receiving plate wells is U-shaped.

11. The multi-well filtration apparatus of claim 9, wherein each of said feeding or receiving plate wells is defined by uniformly downwardly sloping walls, terminating in a nadir located below said associated access port.
